# EUROPEAN PATENT SPECIFICATION

(11) **EP 1 751 106 B1**
(45) Date of publication and mention of the grant of the patent: **29.10.2008**
(21) Application number: 05745442.3
(22) Date of filing: 10.05.2005
(51) Int. Cl.: C07D 209/34, A61K 31/40, A61P 25/00

(54) **PIPERAZINE DERIVATIVES OF ALKYL OXINDOLES**
PIPERAZINDERIVATE VON ALKYLOXINDOLEN
DERIVES DE PIPERAZINE D'OXINDOLES D'ALKYLE

(30) Priority: 11.05.2004 HU 0400954; 05.05.2005 HU 0500461
(43) Date of publication of application: 14.02.2007
(73) Proprietor: EGIS Gyógyszergyár Nyrt, Keresztúri út 30-38 1106 Budapest (HU)
(72) Inventor: VOLK, Balázs, H-1165 Budapest (HU); BARKÓCZY, József, H-1016 Budapest (HU); SIMIG, Gyula, H-1126 Budapest (HU); MEZEI, Tibor, H-1122 Budapest (HU); KAPILLERNÉ DEZSOFI, Rita, H-1055 Budapest (HU); GACSÁLYI, István, H-1201 Budapest (HU); PALLAGI, Katalin, H-1054 Budapest (HU); GIGLER, Gábor, H-1119 Budapest (HU); LÉVAY, György, H-2092 Budakeszi (HU); MÓRICZ, Krisztina, H-1071 Budapest (HU); LEVELEKI, Csilla, H-1029 Budapest (HU); SZIRAY, Nóra, H-1025 Budapest (HU); SZÉNÁSI, Gábor, H-2096 Uröm (HU); EGYED, András, H-1145 Budapest (HU); HÁRSING, László , Gábor, H-1071 Budapest (HU)
(74) Representative: Beszédes, Stephan G.
(86) International application number: PCT/HU2005/000048
(87) International publication number: WO 2005/108363

(56) References cited:
- EP-A- 0 281 309
- EP-A- 0 354 094
- EP-A- 0 376 607
- WO-A-98/08816
- US-A- 4 452 808

## Description

### TECHNICAL FIELD OF THE INVENTION

The invention relates to new, substituted 3-alkyl indol-2-one derivatives and pharmaceutically acceptable acid addition salts thereof, furthermore to a process for the preparation of said compounds. The invention also encompasses pharmaceutical compositions containing said new indol-2-one derivatives and the use of said compounds for the manufacture of medicaments suitable for the treatment of diseases.

More particularly the present invention is concerned with new 3-alkyl indol-2-one derivatives of the general Formula (I), 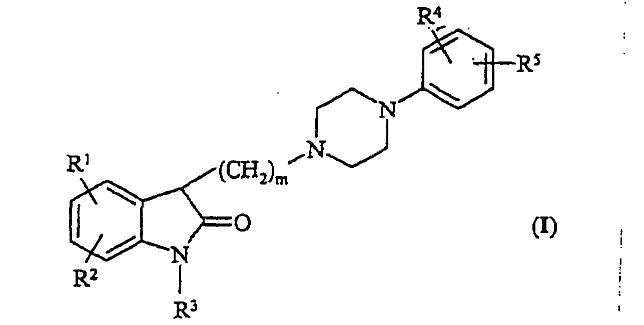 wherein
R¹ represents hydrogen, halogen, alkyl having 1 to 7 carbon atom(s);
R² and R³ represent hydrogen;
R⁴ is hydrogen or halogen;
R⁵ stands for halogen or trifluoromethyl
m is 4;
and pharmaceutically acceptable acid addition salts thereof.

### TECHNICAL BACKGROUND OF THE INVENTION

U.S. patent No. 4,452,808 discloses 4-aminoalkyl indol-2-one derivatives having a selective D₂ receptor activity. These compounds can be used for the treatment of hypertension. One of the compounds provided by this patent, namely 4-[2-(di-N-propylamino)ethyl]-2(3H)-indolone, is used for the clinical treatment of Parkinson disease.

European patent No. 281,309 provides indol-2-one derivatives carrying an arylpiperazinyl-alkyl substituent in position 5, which can be applied for the treatment of psychotic conditions. One of the compounds described in this patent, namely 5-[2-[4-(1,2-benzisothiazol-3-yl)-1-piperazinyl]-ethyl]-6-chloro-1,3-dihydro-2H-indol-2-one, exerts its activity by interaction with D₂, 5-HT_{1A} and 5-HT₂ receptors and is used in the clinical treatment as an antipsychotic agent.

European patent No. 376,607 discloses indol-2-one derivatives substituted in position 3 by an alkylpiperazinyl-aryl group, which exert their activity on 5-HT_{1A} receptors and are useful for the treatment of central nervous disorders.

In the international patent application WO 98/008816 indol-2-one derivatives containing a substituted alkyl-piperazinyl, substituted alkyl-piperidinyl or alkyl-cyclohexyl group in position 3 are disclosed. These compounds possess psychotrophic activity. Said patent specification is completely silent in mentioning anything about the activity profile of the said compounds, and as a field of application only the treatment of depression and anxiety are mentioned.

**From** EP 354 094 A **there have been known indolone derivatives of Formula (A)** 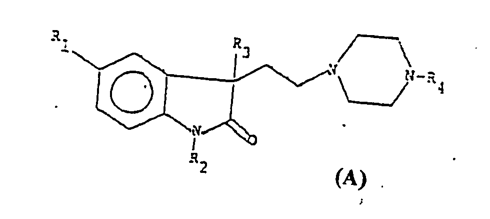 wherein
- R₁: is a hydrogen or halogen atom or a C₁₋₄ alkyl radical,
- R₂: is a hydrogen atom or a C₁₋₄ alkyl radical,
- R₃: is a hydrogen atom or a C₁₋₄ alkyl radical or a S-(C₁₋₄) alkyl radical,
- R₄: is a phenyl, chloro-phenyl, naphthalenyl-1, methoxy-7-naphthalenyl-1, methoxy-6-indanyl-1, methoxy-2--pyridinyl-6, methoxy-3-pyridinyl-2, isoquinoleinyl, methoxy-7--isoquinoleinyl-1, methoxy-7-tetrahydro-1,2,3,4-naphthalenyl-1 or fluoro-7-naphthalenyl-1 radical as well as the diastereoisomers and enantiomers of the compounds (A) and the addition salts of the compounds (A) with pharmaceutically acceptable acids,
and the therapeutic use of these compounds.

Thus in all compounds of the EP 0 354 094 A there is for the linking of the indole and piperazine rings an ethyl group instead of the butyl group (m = 4) of all compounds according to the invention. Thereby many compounds of this reference bind significantly to 5-HT_{1A} receptors as it results from the indication on page 10, line 24 of this reference that the IC₅₀ values are between 0.001 and 0.3 µM which correspond to 1 nM to 300 nM.

Particularly the lower values of this range demonstrate the significant binding of the respective compounds to 5-HT_{1A} receptors. Contrary to this the compounds according to the invention do not bind such strongly to 5-HT_{1A} receptors which binding would have the consequence of unpleasant side-effects which thus are avoided by the compounds according to the invention.

Furthermore the compounds of all detailed examples of this reference contain a naphthalenyl or tetrahydronaphthalenyl group. This group prevents from the binding of the compounds to 5-HT₇ and α₁ receptors and from inhibiting the sinaptosomal serotonin uptake.

The acceleration of technical-social development in the twentieth century constitutes a permanent compulsion of adaptation for humans, which, in adverse cases, my lead to the occurrence of adaptation disorders. Adaptation disorders constitute an important risk factor in the development of diseases of mental or psycho-somatic origin, such as anxiolytic syndrome, stress disorder, depression, schizophrenia, disorders of the sense organs, gastrointestinal diseases, cardiovascular diseases and disorders of the secretory organs.

For the treatment of the above clinical patterns most widespreadly pharmaceuticals exerting their activity on the benzodiazepine system (e.g. diazepam) or on central 5-HT_{1A} receptors (e.g. buspiron, ziprasidon) have been applied. In case of psychosomatic diseases anxiolytic therapy is often complemented by the administration of pharmaceuticals possessing antihypertensive (acting on α₁ or α₂ receptors), or antiulcer (H₁-receptor antagonist) activity.

Anxiolytics of benzodiazepine type are accompanied, however, by several unpleasant side-effects. They have a strong sedative activity, cause decline of the power of concentration and memory and possess muscle relaxant effect. The commercially available active substances possessing anxiolytic activity (buspirone, selective serotonin uptake inhibitors, SSRI's) exert their activity only after a treatment lasting for at least 10 - 14 days. Besides, in the initial period of administration an anxiogenic effect is experienced. Said side-effects influence the quality of life of the patients in an adverse manner and thus restrict the scope of application of such pharmaceuticals.

Beside the stress occurring during adaptation to the environment another great problem of modem society is the rapid ageing of population. Owing to the results of modem medical science life expectancy has increased, and the diseases occurring due to ageing or developing in the declining years, particularly the number of mental diseases has grown in leaps and bounds. The solution of the treatment of Alzheimer's disease, vascular dementias and senile dementia has become a social problem.

As a result of the enumerated processes there is a strong need for new and efficient pharmaceuticals ensuring a more effective treatment of these diseases than those available for the time being.

### SUMMARY OF THE INVENTION

The object of the present invention is to develop pharmaceutical ingredients having more favourable activity profile than those applied for the time being, which are devoid of the above-specified drawbacks and undesired side-effects and which, at the same time, can be used for the treatment and prophylaxis of disorders of the central nervous and cardiovascular system.

The invention is based on the surprising recognition that the **substituted 3-alkyl indol-2-one derivatives of the general Formula (I)** in contrast to the prior art compounds of similar structure - show a considerable binding to both 5-HT₇ and α₁ receptors and considerably inhibit the serotonin sinaptosomal uptake. Accordingly, it can be expected that their applicability will encompass the treatment of both central nervous and cardiovascular disorders.

### DETAILED DESCRIPTION OF THE INVENTION

According to an aspect of the present invention there are provided novel substituted **3-alkyl indol-2-on derivatives of the general** Formula (I), wherein
R¹ represents hydrogen, halogen, alkyl having 1 to 7 carbon atom (s);
R² and R³ represent hydrogen;
R⁴ is hydrogen or halogen;
R⁵ stands for halogen or trifluoromethyl
m is 4 ;
and pharmaceutically acceptable acid addition salts thereof.

The term "alkyl" used throughout this specification is intended to mean straight or branched chain saturated hydrocarbon groups having 1 to 7, preferably 1 to 4 carbon atom(s), (e.g. methyl, ethyl, 1-propyl, 2-propyl, n-butyl, isobutyl or tert. butyl group etc.)

The term "halogen" encompasses the fluorine, chlorine, bromine and iodine atoms and is preferably chlorine or bromine.

The leaving group can be an alkylsulfonyloxy or arylsulfonyloxy group, e.g. methylsulfonyloxy or p-toluenesulfonyloxy group; or a halogen atom, preferably bromine or chlorine.

The term "pharmaceutically acceptable acid addition salts" relates to non-toxic salts of the compounds of the general Formula (I) formed with pharmaceutically acceptable organic or inorganic acids. Inorganic acids suitable for salt formation are e.g. hydrogen chloride, hydrogen bromide, phosphoric, sulfuric or nitric acid. As organic acids formic, acetic, propionic, maleic, fumaric, succinic, lactic, malic, tartaric, citric, ascorbic, malonic, oxalic, mandelic, glycolic, phtalic, benzenesulfonic, p-toluenesulfonic, naphthalic or methanesulfonic acids can be used. Furthermore, carbonates and hydrocarbonates are also considered as pharmaceutically acceptable salts.

To another advantageous subgroup of the compounds of the general Formula (I) belong the compounds wherein R¹ represents hydrogen or halogen; R², R³ and R⁴ stand for hydrogen; R⁵ is halogen; m is 4, and pharmaceutically acceptable acid addition salts thereof.

Particularly advantageous representatives of the compounds of the general Formula (I) are the following derivatives: 3-{4-[4-(4-chlorophenyl)-piperazin-1-yl]-butyl}-6-fluoro-1,3-dihydro-2H-indol-2-one, 3-{4-[4-(4-fluorophenyl)-piperazin-1-yl]-butyl}-1,3-dihydro-2*H*-indol-2-one, 3-{4-[4-(4-chlorophenyl)-piperazin-1-yl]-butyl}-1,3-dihydro-2*H*-indol-2-one, 3-{4-[4-(3-chlorophenyl)-piperazin-1-yl]-butyl}-1,3-dihydro-2*H*-indol-2-one, 3-{4-[4-(3-chlorophenyl)-piperazin-1-yl]-butyl}-5-methyl-1,3-dihydro-2*H*-indol-2-one, 3- {4-[4-(4-chlorophenyl)-piperazin-1-yl]-butyl}-5-methyl-1,3-dihydro-2*H*-indol-2-one, 3-{4-[4-(4-chloro-3-trifluoromethylphenyl)-piperazin-1-yl-butyl}-1,3-dihydro-2*H*-indol-2-one, and pharmaceutically acceptable acid addition salts thereof.

According to a further aspect of the present invention there is provided a process for the preparation of the compounds of the general Formula (I) and pharmaceutically acceptable acid addition salts thereof, which comprises
a./ reacting a compound of the general Formula (II), 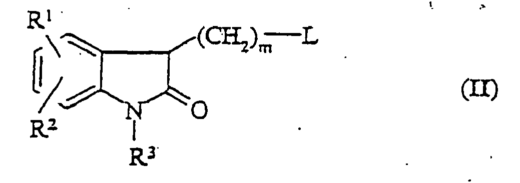 wherein L represents hydroxy, with an aryl-sulfonyl chloride or a straight or branched chain alkylsulfonyl chloride having 1 to 7 carbon atom(s) in the presence of an organic base, and reacting the thus-obtained compound of the general Formula (II), wherein L represents aryl- or alkylsulfonyloxy, with a pyridine derivative of the general Formula (III), 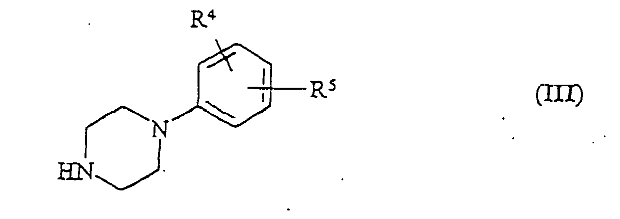 wherein R⁴ and R⁵ are as stated above, in the presence of an acid binding agent, or
b./ reacting a compound of the general Formula (V), 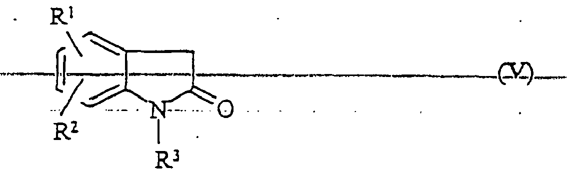
wherein R¹, R² and R³ are as stated above, with a compound of the general Formula (VI), 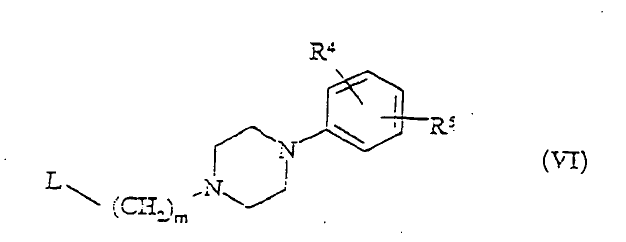 wherein R⁴, R⁵ and m are is stated above and L is an alkylsulfonyloxy or arylsulfonyloxy group or a halogen atom, in the presence of a strong base.

The compounds of the general Formula (I), wherein R¹-R⁵ and m are as stated above, can-be prepared by reacting a compound of the general Formula (II) - wherein R¹-R³, and m are as stated above and L is a leaving group - with a compound of the general Formula (III) - wherein R⁴ and R⁵ are as stated above - according to methods known from the literature [Houben-Weyl: Methoden der organischen Chemie, Georg Thieme Verlag, Stuttgart, 1992, ed. 4, vol. E16d (ed.: D. Klamann); R. C. Larock: Comprehensive Organic Transformations, ed. 2, John Wiley & Sons, New York, 1999, 789; D. A Walsh, Y-H. Chen, J. B. Green, J. C. Nolan, J. M. Yanni J. Med Chem. 1990, 33, 1823-1827].

During the preparation of the compounds of the general Formula (II) the formation of the substituents can be carried out in optional succession according to methods known from the literature. It is expedient to prepare the compounds of the general Formula (II) by reacting a compound of the general Formula (IV)

L-(CH₂)ₘ-L' (IV)

wherein L and m are as stated above, L' is a leaving group or a group that can be converted into a leaving group, with a compound of the general Formula (V), wherein R¹-R³ are as stated above, which has been composed according to methods known from the literature [Houben-Weyl: Methoden der organischen Chemie, Georg Thieme Verlag, Stuttgart, 1977, ed. 4, vol. V/2b; A. R. Katritzky, Ch. W. Rees: Comprehensive Hetero-cyclic Chemistry, ed. 1, Pergamon, Oxford, 1984, vol. 4. (ed.: C. W. Bird, G. W. H. Cheeseman), 98-150 and 339-366; G. M. Karp Org. Prep. Proc. Int. 1993, 25, 481-513; B. Volk, T. Mezei, Gy. Simig Synthesis 2002, 595-597; A. S. Kende, J. C. Hodges Synth. Commun. 1982, 12, 1-10, B. Volk, Gy. Simig Eur. J. Org. Chem. 2003, 18, 3991-3996].

The compounds of the general Formula (I), wherein R¹-R⁵ and m are as stated above, can also be prepared by reacting a compound of the general Formula (V) - wherein R¹-R³ are as stated above - with a compound of the general Formula (VI), wherein R⁴-R⁵ and more as stated above and L is a leaving group - according to methods known from the literature [R. J. Sundberg: The chemistry of indoles, Academic Press, New York, 1970, chapter VII.; A. R. Katritzky, Ch. W. Rees: Comprehensive Heterocyclic Chemistry, 1th Edition, Pergamon, Oxford, 1984, vol. 4 (ed.: C. W. Bird, G. W. H. Cheeseman), 98-150 and 339-366; G. M. Karp Org. Prep. Proc. Int. 1993, 25, 481-513; A. S. Kende, J. C. Hodges Synth. Commun. 1982, 12, 1-10; W. W. Wilkerson, A. A. Kergaye, S. W. Tam J. Med. Chem. 1993, 36, 2899-2907].

The compounds of the general Formula (I), wherein R¹-R⁵ and m are as stated above, can also be prepared by forming the substituents R¹-R⁵ in different succession in the last reaction step. In this case a compound of the general Formula (I) is used as starting substance, wherein all substituents are as stated above except the one to be formed, which can be any one selected from R¹, R², R³, R⁴ and R⁵. The introduction and conversion of the substituents is carried out according to methods known from the literature [Houben-Weyl: Methoden der organischen Chemie, Georg Thieme Verlag, Stuttgart, 1977, 4th Edition, IV/1a-d; vol. V/2b]. During the introduction of the substituents the application or elimination of protecting groups may become necessary. Such methods are specified in T. W. Greene, Protective groups in organic synthesis, John Wiley & Sons, 1981.

The compounds of the general Formulae (III), (IV), (V) and (VI) are known from the literature or can be prepared by analogous methods.

The compounds of the general Formula (I) prepared by the methods according to the invention can be liberated from their salts or converted into pharmaceutically acceptable acid addition salts by methods known from the literature.

According to a further aspect of the present invention there are provided pharmaceutical compositions comprising as active ingredient a compound of the general Formula (I) or a pharmaceutically acceptable acid addition salt thereof in admixture with one or more conventional carrier(s) or auxiliary agent(s).

The pharmaceutical compositions according to the present invention contain generally 0,1-95 % by weight, preferably 1-50 % by weight, particularly 5-30 % by weight of the active ingredient.

The pharmaceutical compositions of the present invention may be suitable for oral (e.g. powders, tablets, coated tablets, capsules, microcapsules, pills, solutions, suspensions or emulsions), parenteral (e.g. injection solutions for intra-venous, intramuscular, subcutaneous or intra-peritoneal use), rectal (e.g. suppositories) transdermal (e.g. plasters) or local (e.g. ointments or plasters) administration or for the application in form of implants. The solid, soft or liquid pharmaceutical compositions according to the invention may be produced by methods conventionally applied in the pharmaceutical industry.

The solid pharmaceutical compositions for oral administration containing the compounds of the general Formula (I) or pharmaceutically acceptable acid addition salts thereof may comprise fillers or carriers (such as lactose, glucose, starch, potassium phosphate, microcrystalline cellulose), binding agents (such as gelatine, sorbite, polyvinyl pyrrolidone), disintegrants (such as croscarmelose, Na-carboxy-methyl cellulose, crospovidone), tabletting auxiliary agents (such as magnesium stearate, talc, polyethylene glycol, silicic acid, silicium dioxide) and surface-active agents (e.g. sodium lauryl sulfate).

The liquid compositions suitable for oral administration can be solutions, suspensions or emulsions. Such compositions can contain suspending agents (e.g. gelatine, carboxymethyl cellulose), emulsifiers (e.g. sorbitane monooleate), solvents (e.g. water, oils, glycerol, propylene glycol, ethanol), buffering agents (e.g. acetate, phosphate, citrate buffers) and preservatives (e.g. methyl-4-hydroxybenzoate).

Liquid pharmaceutical compositions suitable for parenteral administration are generally sterile iotonic solutions optionally containing, in addition to the solvent, buffering agents and preservatives.

Soft pharmaceutical compositions containing as active ingredient a compound of the general Formula (I) or a pharmaceutically acceptable acid addition salt thereof, such as suppositories, contain the active ingredient evenly dispersed in the basic material of the suppository (e.g. in polyethylene glycol or cocoa butter).

According to a further aspect of the present invention there is provided the use of an indol-2-one derivative of the general Formula (I) or a pharmaceutically acceptable acid addition salt thereof for the preparation of pharmaceutical compositions suitable for the treatment or prophylaxis of disorders of the central nervous system or psychosomatic disorders including anxiety syndromes, particularly generalized anxiety disorders, panic disease, compulsive disease, social phobia, agoraphobia, phobias in connection with specific situations, post-traumatic stress disorder, post-traumatic memory disturbances, cognitive disturbances, sexual dysfunction of central nervous system origin, depression, schizophrenia, gastrointestinal diseases and cardiovascular diseases, particularly hypertension.

The pharmaceutical compositions according to the present invention can be prepared by known methods of the pharmaceutical industry. The active ingredient is admixed with pharmaceutically acceptable solid or liquid carriers and/or auxiliary agents and the mixture is brought to galenic form. The carriers and auxiliary agents together with the methods which can be used in the pharmaceutical industry are disclosed in the literature (Remington's Pharmaceutical Sciences, Edition 18, Mack Publishing Co., Easton, USA, 1990).
The pharmaceutical compositions according to the present invention contain generally a dosage unit. The daily dosage for human adults can be generally 0,1-1000 mg/kg body weight of a compound of the general Formula (I) or a pharmaceutically acceptable acid addition salt thereof. Said daily dose can be administered in one or more portion(s). The actual daily dose depends on several factors and is determined by the physician.

According to a further aspect of the present invention there is provided the use of the compounds of the general Formula (I) or pharma-ceutically acceptable acid addition salts thereof for the manufacture of medicaments suitable for the treatment or prophylaxis of disorders of the central nervous system and psychosomatic disorders including anxiety syndrome, particularly generalized anxiety disorders, panic disease, compulsive disease, social phobia, agoraphobia, phobias in connection with specific situations, stress disorder, post-traumatic stress disorder, post-traumatic memory disturbances, cognitive disorder, sexual dysfunction of central nervous system origin, depression, schizophrenia, mental decline caused by cerebral cytolysis, Alzheimer's disease, stroke, dementias, furthermore gastro-intestinal diseases and cardiovascular diseases, particularly hypertension.

The invention is based on the surprising recognition that the 3-alkyl indol-2-on derivatives of the general Formula (I) - in contrary to the prior art compounds of similar structure - show a considerable binding to both 5-HT₇ and α₁ receptors, and inhibit the sinaptosomal serotonin uptake. Such a unique effectivity profile has not so far been described in the literature in connection with any one of the prior art indolone derivatives.

For the determination of 5-HT₇ receptor binding human cloned receptors were used. The α₁ receptor binding was determined from isolated frontal cortex preparation of male Wistar rats weighing 120-200 g. The 5-HT uptake measurements were performed on samples from isolated cortex of male rats. The protein contents of membrane preparations were determined by the method of Lowry (1951).

In the course of 5-HT₇ and α₁ receptor binding studies the ligands were ³H-lizergic acid diethyl amide (LSD) (1.0 nM) and ³H-prazosine (0.3 nM). Clozapine (25 µM) and prazosine (1 µM) were used for the measurement of non-specific bindings. The α₁ binding studies were performed according to the methods of Reader and Greengrass ( Reader, T.A., Briere, R., Grondin, L.: J. Neural Transm. 68, p. 79 (1987); Greengrass, P., Brenner, R.: Eur. J. Pharmacol. 55, p. 323 (1979)). In the serotonin uptake inhibition studies the ligand was tritiated serotonin, the non-specific ligand was fluoxetine (100 µM). On the test results Kᵢ values were calculated. The compounds were considered active if Kᵢ value was less than 100 M/1. The results are shown in Tables 1 to 3.

**Table 1**

| 5-HT₇ receptor binding experiment | |
|---|---|
| No. of Example | Kᵢ M/l |
| 5. | <100 |
| 6. | <100 |
| 7. | <100 |
| 8. | <100 |
| 9. | <100 |
| 10. | <100 |
| 11. | <100 |
| 12. | <100 |
| 14. | <100 |
| 15. | <100 |
| 16. | <100 |
| 17. | <100 |
| 18. | <100 |

**Table 2**

| α₁ receptor binding experiment | |
|---|---|
| No. of Example | Kᵢ M/l |
| 5. | <100 |
| 6. | <100 |
| 7. | <100 |
| 8. | <100 |
| 9. | <100 |
| 11. | <50 |
| 12. | <100 |
| 17. | <50 |
| 18. | <50 |

**Table 3**

| Inhibition of serotonin uptake | |
|---|---|
| No. of Example | Kᵢ M/l |
| 8. | <100 |
| 17. | <100 |
| 18. | <100 |

From the results of the above Tables 1 to 3 it can be established that the test compounds exhibit considerable affinity to both 5-HT₇ and α₁ receptors and considerably inhibit the sinaptosomal serotonin uptake.

On the basis of the above pharmacological experiments it can be established that the compounds according to the invention have a valuable profile of action rendering them suitable for the treatment or prophylaxis of mental and cardiovascular diseases including e.g. depression, anxiety, compulsive disease, panic disease, social phobia, schizophrenia, mood disorders, mania, mental decline, stroke, cell death in certain areas of the central nervous system, neurodegeneration caused by mental decline, Alzheimer's disease, dementia, post-traumatic disease, stress disease, disorders of the cardiovascular system, particularly hyper-tension.

Further details of the present invention are provided in the following examples without limiting the scope of protection to said examples.

### Preparation of mesyl esters (process "A")

The 3-(4-hydroxybutyl)-oxindoles are prepared according to a method known from the literature [B. Volk, T. Mezei, Gy. Simig Synthesis 2002, 595; B. Volk, Gy. Simig Eur. J. Org. Chem. 2003, 18, 3991-3996].
55 mmoles of 3-(4-hydroxybutyl)-oxindol are dissolved in 150 ml of THF, 15.2 ml (110 mmoles) of triethyl amine are added to it, and the solution is cooled in an acetone-dry ice bath to -78 °C. While stirring at the same temperature 8.5 ml (110 mmoles) of mesyl chloride are dropped to it and the solution is allowed to warm to room temperature. It is stirred at room temperature for 1 hour, the triethyl amine hydrochloride is filtered off, the filtrate is evaporated, the residue is taken up in ethyl acetate and extracted several times with 10 % by volume hydrogen chloride solution until the pH of the aqueous phase has become acidic. The organic phase is dried over sodium sulfate, evaporated, the residual oil is crystallized by trituration with diisopropyl ether, stirred in 100 ml of diisopropyl ether, filtered, washed with hexane and dried. The product is purified by recrystal-lization from the solvent indicated after the melting point of the given substance.

### Example 1

### does not relate to the claimed subject-matter but to the preparation of a starting substance

### 3-(4-Mesyloxybutyl)-1,3-dihydro-2H-indol-2-one

The title compound is prepared according to process A starting from 3-(4-hydroxybutyl)-1,3-dihydro-2*H*-indol-2-one.
M.p.: 84-85 °C (heptane-ethyl acetate).

IR (KBr): 3180, 1705 (C=O) cm⁻¹.

¹H-NMR (CDCl₃, TMS, 400 MHz): 9.33 (1H, s), 7.22 (1H, d, *J* = 7.1 Hz), 7.21 (1H, t, *J* = 7.0 Hz), 7.03 (1H, t, *J* = 7.5 Hz), 6.93 (1H, d, *J* = 7.6 Hz), 4.19 (2H, t, *J* = 6.5 Hz), 3.49 (1H, t, *J* = 6.0 Hz), 2.97 (3H, s), 2.05-1.98 (2H, m), 1.82-1.72 (2H, m) 1.58-1.40 (2H, m) ppm.

¹³C-NMR (CDCl₃, TMS, 101 MHz): 180.5, 141.6, 129.1, 127.9, 123.9, 122.3, 109.9, 69.5, 45.7, 37.2, 29.6, 28.9, 21.6 ppm.

### Example 2

### does not relate to the claimed subject-matter but to the preparation of a starting substance

### 5-Fluoro-3-(4-mesyloxybutyl)-1,3-dihydro-2H-indol-2-one

The title compound is prepared according to process A starting from 5-fluoro-3-(4-hydroxy-butyl)-1,3-dihydro-2*H*-indol-2-one.
M.p.: 106-108 °C (hexane-ethyl acetate).

IR (KBr): 3169, 1702 (C=O), 1356, 1175 (SO₂) cm⁻¹.

¹H-NMR (CDCl₃, TMS, 500 MHz): 1.43-1.55 (2H, m), 1.73-1.83 (2H, m), 1.97-2.05 (2H, m), 2.99 (3H, s), 3.50 (1H, *J* = 5.9 Hz), 4.21 (2H, dq, *J* = 1.4, 6.3 Hz), 6.86 (1H, dd, *J* = 4.3, 8.4 Hz), 6.93 (1H, dt, *J* = 2.3, 9.0 Hz), 6.97 (1H, dd, J = 2.0, 7.3 Hz), 9.22 (1H, s) ppm.

¹³C-NMR (CDCl₃, TMS, 125.6 MHz): 180.2, 158.9 (d, *J* = 240.6 Hz), 137.5 (d, *J* = 1.7 Hz), 130.8 (d, *J* = 8.5 Hz), 114.3 (d, *J* = 27.5 Hz), 111.9 (d, *J* = 24.8 Hz), 110.4 (d, *J* = 8.1 Hz), 69.4, 46.2, 37.3, 29.5, 28.9, 21.5 ppm.

### Example 3

### does not relate to the claimed subject-matter but to the preparation of a starting substance

### 6-Fluoro-3-(4-mesyloxybutyl)-1,3-dihydro-2H-indol-2-one

The title compound is prepared according to process A starting from 6-fluoro-3-(4-hydroxy-butyl)-1,3-dihydro-2*H*-indol-2-one.
M.p.: 105-108 °C (hexane-ethyl acetate).

IR (KBr): 3161, 1705 (C=O), 1335, 1313, 1167 (SO₂) cm⁻¹.

¹H-NMR (CDCl₃, TMS, 500 MHz): 1.46-1.51 (2H, m), 1.78 (2H, kv, *J* = 6.7 Hz), 2.00 (2H, q, *J* = 8.1 Hz), 2.99 (3H, s), 3.46 (1H, t, *J* = 5.9 Hz), 4.21 (2H, dt, *J* = 1.5, 6.5 Hz), 6.68 (1H, dd, *J* = 2.3, 8.8 Hz), 6.72 (1H, dt, *J* = 2.3, 8.9 Hz), 7.15 (1H, dd, *J* = 5.4, 8.1 Hz), 9.15 (1H, br s) ppm.

¹³C-NMR (CDCl₃, TMS, 125.6 MHz): 21.6, 28.9, 29.7, 37.3, 45.3, 69.5, 98.6 (d, *J* = 27.4 Hz), 108.7 (d, *J* = 22.5 Hz), 124.5 (d, *J* = 3.0 Hz), 124.9 (d, *J* = 9.5 Hz), 142.8 (d, *J* = 11.8 Hz), 162.6 (d, *J* = 244.6 Hz), 180.7 ppm.

### Example 4

### does not relate to the claimed subject-matter but to the preparation of a starting substance

### 5-Methyl-3-(4-mesyloxybutyl)-1,3-dihydro-2H-indol-2-one

The title compound is prepared according to process A starting from 3-(4-hydroxybutyl)-5-methyl-1,3-dihydro-2*H*-indol-2-one.
M.p.: 89-90 °C (hexane-ethyl acetate).

IR (KBr): 3175, 1710 (C=O), 1351, 1176 (SO₂) cm⁻¹.

¹H-NMR (CDCl₃, TMS, 400 MHz): 9.13 (1H, s), 7.03 (1H, s), 7.01 (1H, dd, *J* = 7.9, 0.8 Hz), 6.81 (1H, d, *J* = 7.9 Hz), 4.20 (2H, t, *J* = 6.5 Hz), 3.45 (1H, t, *J* = 5.9 Hz), 2.98 (3H, s), 2.33 (3H, s), 1.99 (2H, q, *J* = 7.4 Hz), 1.79-1.75 (2H, m), 1.51-1.42 (2H, m) ppm.

¹³C-NMR (CDCl₃, TMS, 101 MHz): 180.4, 139.1, 131.7, 129.2, 128.2, 124.7, 109.5, 69.6, 45.8, 37.2, 29.6, 28.9, 21.5, 21.0 ppm.

### Coupling reaction of mesyl esters with bases (process "B")

In the coupling reaction the appropriate mesyl ester is coupled to the secondary amine. The melt of the secondary amine (12 mmoles) is warmed to 120 °C under slow stirring, and the mesyl compound (12 mmoles) and sodium carbonate (1.36 g; 12 mmoles) are added to it at the same temperature. The mixture is allowed to react for 1 hour, the melt is allowed to cool, ethyl acetate and water are added to it and the phases are separated. The organic phase is evaporated, and the residual oil is subjected to chromatography on a short column using ethyl acetate as eluent. As main products the desired compounds are obtained.
Processing method 1: If the product purified by column chromatography gets crystalline upon rubbing with diethyl ether, it is filtered off and recrystallized from a mixture of hexane and ethyl acetate. The desired compounds are obtained in form of white crystals.
Processing method 2: If the basic product does not get crystalline upon the addition of diethyl ether, it is dissolved in 200 ml of ether, the slight amount of floating precipitate is filtered off and to the pure solution the calculated amount (1 molar equivalent) of hydrogen chloride dissolved in ether diluted with 50 ml of diethyl ether is dropped under vigorous stirring. The separated white salt is filtered off, washed with ether and hexane and dried in a vacuum pistol at room temperature for 3 hours.
Processing method 3: If the basic product does not get crystalline upon the addition of diethyl ether and does not provide a well-filterable salt with hydrogen chloride, it is dissolved in 100 ml of hot ethyl acetate, and a solution of 1 molar equivalent of oxalic acid dihydrate in 30 ml of hot ethyl acetate is dropped to it within 10 minutes, under stirring. The white oxalate salt gets separated upon cooling. It is filtered off at room temperature, washed with ethyl acetate and hexane and dried.

### Example 5

### 3-{4-[(3-Chlorophenyl)-piperazin-1-yl]-butyl}-6-fluoro-1,3-dihydro-2H-indol-2-one monooxalate

The title compound is prepared according to process "B" by applying processing method 3 starting from 6-fluoro-3-(4-mesyloxybutyl)-1,3-dihydro-2*H*-indol-2-one and 1-(3-chlorophenyl)-piperazine.
M.p.: 217-219 °C.

IR (KBr): 3256, 1712 (C=O), 1626, 1139 cm⁻¹.

¹H-NMR (DMSO-*d₆*, TMS, 400 MHz): 1.29-1.26 (2H, m), 1.65-1.58 (2H, m), 1.89-1.80 (2H, m), 2.88 (2H, t, *J* = 7.9 Hz), 3.08 (4H, br s), 3.38 (4H, br s), 3.44 (1H, t, *J* = 5.4 Hz), 6.65 (1H, dd, *J* = 2.4, 9.1 Hz), 6.75 (1H, dt, *J* = 2.4, 9.1 Hz), 6.89 (1H, dd, *J* = 1.3, 7.8 Hz), 6.94 (1H, dd, *J* = 1.8, 8.4 Hz), 7.01 (1H,t, *J =* 2.1 Hz), 7.24 (1H, t, *J* = 8.2 Hz), 7.29-7.22 (1H, m), 10.5 (1H, s) ppm.

¹³C-NMR (DMSO-*d*₆, TMS, 101 MHz): 22.7, 24.0, 29.6, 44.6, 45.7, 51.0, 55.8, 97.6 (d, *J* = 27.1 Hz), 107.4 (d, *J* = 21.7 Hz), 114.2, 115.2, 119.1, 125.4 (d, *J* = 15.3 Hz), 125.5(d, *J* = 7.4 Hz), 130.7, 134.1, 144.5 (d, *J* = 12.2 Hz), 151.4, 162.1 (d, *J* = 240.7 Hz), 164.4, 179.4 ppm.

| Elementary analysis for the Formula C₂₄H₂₇ClFN₃O₅ (491.95): | | | | |
|---|---|---|---|---|
| Calculated: | C 58.60, | H 5.53, | Cl 7.21, | N 8.54 % |
| Found: | C 58.48, | H 5.52, | Cl 7.11, | N 8.50 %. |

### Example 6

### 3-{4-[4-(3-Chlorophenyl)-piperazin-1-yl]-butyl}-5-fluoro-1,3-dihydro-2H-indol-2-one monohydrochloride

The title compound is prepared according to process "B" by applying processing method 2 starting from 5-fluoro-3-(4-mesyloxybutyl)-1,3-dihydro-2*H*-indol-2-one and 1-(3-chlorophenyl)-piperazine.
M.p.: 180-184 °C.

IR (KBr): 3421, 3145, 1712 (C=O), 1189, 778 cm⁻¹.

¹H-NMR (DMSO-*d₆*, TMS, 400 MHz): 1.33-1.24 (2H, m), 1.93-1.70 (4H, m), 3.16-3.03 (4H, m), 3.18 (2H, m), 3.38 (1H, m), 3.49 (2H, d, *J* = 12.4 Hz), 3.86 (2H, d, *J* = 12.9 Hz), 6.81 (1H, dd, *J* = 4.5, 8.4 Hz), 6.87 (1H, dd, *J* = 7.9, 1.4 Hz), 6.96 (1H, dt, *J* = 2.1, 8.4 Hz), 7.00 (1H, m), 7.05 (1H, t, *J* = 2.0 Hz), 7.21 (1H, dd, *J* = 2.0, 8.5 Hz), 7.25 (1H, t, *J* = 8.2 Hz), 10.4 (1H, s, 10.9 (1H, br s) ppm.

¹³C-NMR (DMSO-*d₆*, TMS, 101 MHz): 22.5, 23.1, 29.3, 45.0, 45.5 (d, *J* = 1.5 Hz), 50.4 , 55.2, 109.9 (d, *J* = 8.0 Hz), 111.2 (d, *J* = 24.4 Hz), 114.0 (d, *J* = 23.3 Hz), 114.3, 115.4, 119.4, 130.8, 131.6 (d, *J* = 8.4 Hz), 134.1, 139.1 (d, *J* = 1.5 Hz), 151.0, 158.1 (d, *J* = 236.1 Hz), 178.8 ppm.

| Elementary analysis for the Formula C₂₂H₂₆Cl₂FN₃O (438.38): | | | |
|---|---|---|---|
| Calculated: | H 5.98, | Cl 16.17, | N 9.59 %. |
| Found: | H 6.26, | Cl 15.50, | N 9.17 %. |

### Example 7

### 3- {4-[4-(4-Chlorophenyl)-piperazin-1-yl]-butyl}-5-fluoro-1,3-dihydro-2H-indol-2-one monohydrochloride

The title compound is prepared according to process "B" by applying processing method 2 starting from 5-fluoro-3-(4-mesyloxybutyl)-1,3-dihydro-2*H*-indol-2-one and 1-(4-chlorophenyl)-piperazine.
M.p.: 193-196 °C.

IR (KBr): 3145, 1712 (C=O), 821 cm⁻¹.

¹H-NMR (DMSO-*d₆*, TMS, 400 MHz): 1.32-1.22 (2H, m), 1.95-1.71 (4H, m), 3.20-3.06 (6H, m), 3.53-3.49 (3H, m), 3.80-3.76 (2H, m), 6.82 (1H, dd, *J* = 4.5, 8.5 Hz), 7.04-6.98 (1H, m), 7.01 (2H, d, *J* = 9.1 Hz), 7.21 (1H, dd, *J* = 2.1, 8.6 Hz), 7.28 (2H, d, *J* = 9.1 Hz), 10. 5 (1H, s), 11.1 (1H, br s) ppm.

¹³C-NMR (DMSO-*d*₆, TMS, 101 MHz): 22.5, 23.1, 29.3, 42.6, 45.3, 45.4 , 45.6 (d, *J* = 1.9 Hz), 50.5, 55.1, 110.0 (d, *J* = 8.4 Hz), 112.1 (d, *J* = 24.4 Hz), 114.0 (d, *J* = 23.3 Hz), 117.6, 117.7, 123.7, 129.0, 131.6 (d, *J* = 8.4 Hz), 139.2 (d, *J* = 1.9 Hz), 148.7, 149.1, 158.1 (d, *J* = 235.8 Hz), 178.8 ppm.

| Elementary analysis for the Formula C₂₂H₂₆Cl₂FN₃O (438.38): | | | | |
|---|---|---|---|---|
| Calculated: | C 60.28, | H 5.98, | Cl 16.17, | N 9.59 %. |
| Found: | C 59.35, | H 5.93, | Cl 16.44, | N 9.46 %. |

### Example 8

### 3- {4-[4-(4-Chlorophenyl)-piperazin-1-yl]-butyl}-6-fluoro-1,3-dihydro-2H-indol-2-one monohydrochloride

The title compound is prepared according to process "B" by applying processing method 2 starting from 6-fluoro-3-(4-mesyloxybutyl)-1,3-dihydro-2*H*-indol-2-one and 1-(4-chlorophenyl)-piperazine.
M.p.: 149-151 °C.

IR (KBr): 3148, 2586, 2459, 1716 (C=O) cm⁻¹.

¹H-NMR (DMSO-*d*₆, TMS, 400 MHz): 1.31-1.07 (2H, m), 1.91-1.66 (4H, m), 3.21-2.99 (6H, m), 3.51 (1H, t, *J* = 5.9 Hz), 3.65-3.60 (2H, m), 3.81-3.78 (2H, m), 6.69-6.64 (1H, m), 6.80-6.72 (1H, m), 7.03-6.99 (2H, m), 7.32-7.26 (3H, m), 10.6-10.5 (1H, s), 11.2 (1H, br s) ppm.

¹³C-NMR (CD₃OD, TMS, 101 MHz): 182.2, 164.4 (d, *J* = 243.0 Hz), 150.0, 144.7 (d, *J* = 12.2 Hz), 130.3, 128.6, 127.3, 126.5 (d, *J* = 9.5 Hz), 119.5, 109.4 (d, *J* = 22.5 Hz), 99.2 (d, *J* = 27.5 Hz), 57.8, 53.1, 48.0, 46.7, 30.7, 25.0, 21.6 ppm.

| Elementary analysis for the Formula C₂₂H₂₆Cl₂FN₃O (438.38): | | | |
|---|---|---|---|
| Calculated: | C H 5.98, | Cl 16.17, | N 9.59 %. |
| Found: | C H 5.94, | Cl 15.51, | N 9.16 %. |

### Example 9

### 3- {4-[4-(3-Chlorophenyl)-piperazin-1-yl]-butyl}-5-methyl-1,3-dihydro-2H-indol-2-one

The title compound is prepared according to process "B" by applying processing method 1 starting from 5-methyl-3-(4-mesyloxybutyl)-1,3-dihydro-2*H*-indol-2-one and 1-(3-chlorophenyl)-piperazine.
M.p.: 122-124 °C (hexane-EtOAc).

IR (KBr): 3174 (NH), 1690 (C=O) cm⁻¹.

¹H-NMR (CDCl₃, TMS, 400 MHz): 1.58-1.38 (4H, m), 2.00-1.95 (2H, m), 2.33 (3H, s), 2.36 (2H, t, *J* = 7.6 Hz), 2.54 (4H, t, *J* = 5.1 Hz), 3.17 (4H, t, *J* = 5.1 Hz), 3.44 (1H, t, *J* = 6.0 Hz), 6.76 (1H, ddd, *J* = 0.7, 2.5, 8.2 Hz), 6.78 (1H, d, *J* = 8.1 Hz), 6.79(1H, ddd, *J* = 0.8, 1.9, 7.8 Hz), 6.85 (1H, t, *J* = 2.1 Hz), 7.01 (1H, d, *J* = 7.9 Hz), 7.04 (1H, d, *J* = 0.5 Hz), 7.14 (1H, t, *J* = 8.2 Hz), 8.78 (1H, s) ppm.
¹³C-NMR (CDCl₃, TMS, 101 MHz): 180.5, 152.3, 139.1, 134.9, 131.7, 129.9, 129.7, 128.1, 124.9, 119.1, 115.6, 113.7, 109.3, 58.2, 52.9, 48.5, 46.0, 30.4, 26.7, 23.7, 21.1 ppm.

| Elementary analysis for the Formula C₂₃H₂₈ClN₃O (397.95): | | | | |
|---|---|---|---|---|
| Calculated: | C 69.42, | H 7.09, | Cl 8.91, | N 10.56 %. |
| Found: | C 69.29, | H 7.12, | Cl 8.69, | N 10.51 %. |

### Example 10

### 3-{4-[4-(4-Chlorophenyl)-piperazin-1-yl]-butyl}-5-methyl-1,3-dihydro-2H-indol-2-one

The title compound is prepared according to process "B" by applying processing method 1 starting from 5-methyl-3-(4-mesyloxybutyl)-1,3-dihydro-2*H*-indol-2-one and 1-(4-chlorophenyl)-piperazine.
M.p.: 159-161 °C (EtOAc).

IR (KBr): 3186, 1702 (C=O) cm⁻¹.

¹H-NMR (CDCl₃, TMS, 400 MHz): 1.58-1.37 (4H, m), 2.02-1.95 (2H, m), 2.32 (3H, s), 2.55 (4H, t, *J* = 5.0 Hz), 3.13 (4H, t, *J* = 5.0 Hz), 3.44 (1H, t, *J* = 5.9 Hz), 6.78 (1H, d, *J* = 7.8 Hz), 6.81 (2H, d, *J* = 9.1 Hz), 6.99 (1H, d, J = 7.9 Hz), 7.04 (1H, s), 7.18 (2H, d, *J* = 9.1 Hz), 8.88 (1H, s) ppm.

¹³C-NMR (CDCl₃, TMS, 101 MHz): 180.5, 149.9, 139.1, 131.6, 129.7, 128.8, 128.1, 124.8, 124.4, 117.1, 109.3, 58.2, 53.0, 49.0, 46.0, 30.3, 26.7, 23.7, 21.1 ppm.

| Elementary analysis for the Formula C₂₃H₂₈ClN₃O (397.95): | | | | |
|---|---|---|---|---|
| Calculated: | C 69.42, | H 7.09, | Cl 8.91, | N 10.56 %. |
| Found: | C 68.76, | H 7.10, | Cl 8.80, | N 10.54 %. |

### Example 11

### 3- {4-[4-(4-Fluorophenyl)-piperazin-1-yl]-butyl}-1,3-dihydro-2H-indol-2-one

The title compound is prepared according to process "B" by applying processing method 1 starting from 3-(4-mesyloxybutyl)-1,3-dihydro-2*H*-indol-2-one and 1-(4-fluorophenyl)-piperazine.
M.p.: 113-115 °C (hexane-EtOAc).

IR (KBr): 3192, 1720 (C=O) cm⁻¹.

¹H-NMR (CDCl₃, TMS, 400 MHz): 1.57-1.36 (4H, m), 2.04-1.95 (2H, m), 2.36 (2H, t, *J* = 7.5 Hz), 2.56 (4H, t, *J* = 5.0 Hz), 3.09 (4H, t, *J* = 5.0 Hz), 3.37 (1H, t, *J* = 5.9 Hz), 6.85 (2H, dd, *J* = 4.6, 9.2 Hz), 6.89 (1H, d, *J* = 7.7 Hz), 6.94 (2H, t, *J* = 8.8 Hz), 7.01 (1H, dt, *J* = 0.9, 7.5 Hz), 7.19 (1H, d, *J* = 7.7 Hz), 7.21 (1H, t, *J* = 6.7 Hz), 9.33 (1H, s) ppm.

¹³C-NMR (CDCl₃, TMS, 101 MHz): 23.7, 26.7, 30.3, 46.0, 50.0, 53.1, 58.2, 109.7, 115.4 (d, *J* = 22.1 Hz), 117.6 (d, *J* = 7.6 Hz), 122.1, 124.0, 127.8, 130.0, 141.7, 147.9, 157.0 (d, *J* = 238.4 Hz), 180.7 ppm.

Elementary analysis for the Formula C₂₂H₂₆FN₃O (367.47):
Calculated: C 71.91, H 7.13, F 5.17, N 11.43 %.
Found: C 71.12, H 7.32, N 11.28 %.

### Example 12

### 3- {4-[4-(3,4-Dichlorophenyl)-piperazin-1-yl]-butyl}-1,3-dihydro-2H-indol-2-one

The title compound is prepared according to process "B" by applying processing method 1 starting from 3-(4-mesyloxybutyl)-1,3-dihydro-2*H*-indol-2-one and 1-(3,4-dichlorophenyl)-piper-azine.
M.p.: 112-114 °C (hexane-EtOAc).

IR (KBr): 3175, 1718 (C=O) cm⁻¹.

¹H-NMR (CDCl₃, TMS, 400 MHz): 1.47-1.36 (2H, m), 1.60-1.56 (2H, m), 2.04-1.96 (2H, m), 2.44 (2H, t, *J* = 7.1 Hz), 2.63 (4H, m), 3.20 (4H, m), 3.47 (2H, t, *J* = 5.9 Hz), 6.71 (1H, dd, *J* = 2.8, 9.0 Hz), 6.91 (1H, d, *J* = 9.2 Hz), 6.92 (1H, d, *J* = 2.9 Hz), 7.02 (1H, t, *J* = 7.5 Hz), 7.26-7.18 (3H, m) , 9.02 (1H, s) ppm.

¹³C-NMR (CDCl₃, TMS, 101 MHz): 180.4, 150.3, 141.6, 132.7, 130.4, 129.5, 127.9, 124.0, 122.2, 120.6, 117.3, 115.3, 109.7, 57.9, 48.2, 45.9, 45.9, 30.1, 26.2, 23.5 ppm.

| Elementary analysis for the Formula C₂₂H₂₅Cl₂N₃O (418.37): | | | | |
|---|---|---|---|---|
| Calculated: | C 63.16, | H 6.02, | Cl 16.95, | N 10.04 %. |
| Found: | C 63.04, | H 6.02, | Cl 16.78, | N 10.01 %. |

### Example 13

### 3-{4-[4-(3-Chloro-4-fluorophenyl)-piperazin-1-yl]-butyl}-1,3-dihydro-2H-indol-2-one monohydrochloride

The title compound is prepared according to process "B" by applying processing method 2 starting from 3-(4-mesyloxybutyl)-1,3-dihydro-2*H*-indol-2-one and 1-(3-chloro-4-fluoro-phenyl)-piperazine.
M.p.: 180-183 °C.

IR (KBr): 3432, 1709 (C=O), 735 cm⁻¹.

¹H-NMR (DMSO-*d*₆, TMS, 400 MHz): 1.36-1.23 (2H, m), 1.94-1.72 (4H, m), 3.15-3.04 (4H, m), 3.19 (2H, t, *J*= 12.0 Hz), 3.51-3.44 (3H, m), 3.78 (2H, d, *J* = 12.2 hz), 6.85 (1H, d, *J*= 7.7 Hz), 7.02-6.93 (2H, m), 7.22-7.15 (2H, m), 7.32-7.26 (2H, m), 10.43 81H, s), 11.2 (1H, s) ppm.

¹³C-NMR (DMSO-*d*₆, TMS, 101 MHz): 22.7, 23.1, 29.6, 45.0, 45.6, 50.4, 55.2, 109.4, 116.3 (d, *J* = 6.5 hz), 117.1 (d, *J* = 21.4 Hz), 117.6, 119.9 (d, *J* = 18.3 Hz), 121.4, 124.2, 127.8, 129.7, 142.9, 147.2, 151.5 (d, *J* = 239.2 Hz), 178.9 ppm.

Elementary analysis for the Formula C₂₂H₂₆Cl₂FN₃O (438.38):
Calculated: C 60.28, H 5.98, Cl 16.17, N 9.59 %.
Found: C 59.60, H 6.06, Cl 15.85, N 9.32 %.

### Example 14

### 3-{4-[4-(4-Chloro-3-trifluoromethylphenyl)-piperazin-1-yl]-butyl}-1,3-dihydro-2H-indol-2-one monohydrochloride

The title compound is prepared according to process "B" by applying processing method 2 starting from 3-(4-mesyloxybutyl)-1,3-dihydro-2*H*-indol-2-one and 1-(4-chloro-3-trifluoromethyl-phenyl)-piperazine.
M.p.: 123-125 °C.

IR (KBr): 3150, 1709 (C=O), 2554, 2462, 1129 cm⁻¹.

¹H-NMR (DMSO-*d*₆, TMS, 400 MHz): 1.34-1.26 (2H, m), 1.94-1.74 (4H, m), 3.09-3.06 (4H, m), 3.30 (2H, t, *J* = 12.3 Hz), 3.64-3.47 (3H, m), 3.94 (2H, d, *J* = 12.6 Hz), 6.86 (1H, d, *J* = 7.6 Hz), 6.96 (1H, t, *J* = 7.4 Hz), 7.18 (1H, t, *J* = 7.6 Hz), 7.28 (1H, d, *J* = 7.6 Hz), 7.35 (1H, d, *J* = 2.2 Hz), 7.36-7.27 (1H, m), 7.54 (1H, d, *J* = 8.8 Hz), 10.44 (1H, s), 11.30 (1H, br s) ppm.
¹³C-NMR (DMSO-*d*₆, TMS, 101 MHz): 22.7, 23.1, 29.6, 44.8, 45.0, 50.2, 55.1, 109.4, 114.4 (q, *J* = 5.0 Hz), 120.0 (q), 120.5, 121.4, 123.1 (q, *J* = 273.1 Hz), 124.2, 127.2 (q, *J* = 30.1 Hz), 127.8, 129.7, 132.3, 142.9, 148.6, 178.9 ppm.

Elementary analysis for the Formula C₂₃H₂₆Cl₂F₃N₃O (488.38):
Calculated: C 56.57, H 5.37, Cl 14.52, F 11.67, N 8.60 %.
Found: C 55.88, H 5.45,Cl 14.44, N 8.59 %.

### Example 15

### 5-Fluoro-3-{4-[4-(4-fluorophenyl)-piperazin-1-yl]-butyl}-1,3-dihydro-2H-indol-2-one

The title compound is prepared according to process "B" by applying processing method 1 starting from 5-Huoro-3-(4-mesyloxybutyl)-1,3-dihydro-2*H*-indol-2-one and 1-(4-fluorophenyl)-piperazine.
M.p.: 135-137 °C (hexane-EtOAc).

IR (KBr): 3169, 1703 (C=O) cm⁻¹.

¹H-NMR (DMSO-*d*₆, TMS, 400 MHz): 1.35-1.16 (2H, m), 1.46-1.35 (2H, m), 1.96-1.77 (2H, m), 2.26 (2H, t, *J* = 6.9 Hz), 2.44 (4H, t, *J* = 4.8 Hz), 3.02 (4H, t, *J* = 4.8 Hz), 3.48 (1H, t, *J* = 5.6 Hz), 6.79 (1H, dd, *J* = 4.5, 8.4 Hz), 6.92 (2H, dd, *J* = 4.8, 9.3 Hz), 7.03 (2H, t, *J* = 8.9 Hz), 7.06-6.96 (1H, m), 10.36 (1H, s) ppm.

¹³C-NMR (DMSO-*d*₆, TMS, 101 MHz): 23.1, 26.3, 29.5, 45.8, 49.1, 52.8, 57.6, 109.8 (d, *J* = 8.4 Hz), 112.0 (d, *J* = 24.4 Hz), 113.8 (d, *J* = 22.9 Hz), 115.4 (d, *J* = 22.1 Hz), 131.8 (d, *J* = 8.4 Hz), 139.1, 148.1, 156.1 (d, *J* = 235.4 Hz), 158.0 (d, *J* = 235.7 Hz), 179.0 ppm.

| Elementary analysis for the Formula C₂₂H₂₅F₂N₃O (385.46): | | |
|---|---|---|
| Calculated: | H 6.54, | N 10.90 %. |
| Found: | H 6.67, | N 10.40 %. |

### Example 16

### 3-{4-[4-(4-Chlorophenyl)-pipemin-1-yl]-butyl}-1,3-dihydro-2H-indol-2-one

The title compound is prepared according to process "B" by applying processing method 1 starting from 3-(4-mesyloxybutyl)-1,3-dihydro-2*H*-indol-2-one and 1-(4-chlorophenyl)-piperazine.

M.p.: 136-137 °C (hexane-EtOAc).

IR (KBr): 3203, 1718 (C=O), 754 cm⁻¹.

¹H-NMR (CDCl₃, TMS, 400 MHz): 1.58-1.36 (4H, m), 2.05-1.96 (2H, m), 2.36 (2H, t, *J =* 7.5 Hz), 2.55 (4H, t, *J* = 5.0 H), 3.13 (4H, t, *J* = 5.0 Hz), 3.48 (1H, t, *J* = 6.0 H), 6.81 (2H, d, *J* = 9.2 Hz), 6.88 (1H, d, *J* = 7.7 Hz), 7.02 (1H, dt, *J* = 0.9, 7.6 Hz), 7.18 (2H, d, *J* = 9.1 Hz), 7.24-7.15 (2H, m), 8.60 (1H, s) ppm.

¹³C-NMR (CDCl₃, TMS, 101 MHz): 180.3, 149.9, 141.5, 129.7, 128.8, 127.8, 124.4, 124.1, 122.2, 117.1, 109.6, 58.2, 53.0, 49.1, 45.9, 30.4, 26.8, 23.7 ppm.

| Elementary analysis for the Formula C₂₂H₂₆ClN₃O (383.93): | | | | |
|---|---|---|---|---|
| Calculated: | C 68.83, | H 6.83, | Cl 9.23 | N 10.94 %. |
| Found: | C 68.49, | H 6.89, | Cl 9.08, | N 10.81 %. |

### Example 17

### 3-{4-[4-(3-Chlorophenyl)-piperazin-1-yl]-butyl}-1,3-dihydro-2H-indol-2-one

The title compound is prepared according to process "B" by applying processing method 1 starting from 3-(4-mesyloxybutyl)-1,3-dihydro-2*H*-indol-2-one and (3-chlorophenyl)-piperazine.
M.p.: 112-114 °C (hexane-EtOAc).

IR (KBr): 3200, 1715 (C=O), 750 cm⁻¹.

¹H-NMR (CDCl₃, TMS, 400 MHz): 1.57-1.38 (4H, m), 2.03-1.97 (2H, m), 2.35 (2H, t, *J* = 7.5 Hz), 2.54 (4H, t, *J* = 5.0 Hz), 3.16 (4H, t, *J* = 5.0 Hz), 3.48 (1H, t, *J* = 5.9 Hz), 6.75 (1H, ddd, *J* = 0.6, 2.4, 8.4 Hz), 6.78 (1H, ddd, *J* = 0.7, 1.9, 7.8 Hz), 6.85 (1H, t, *J* = 2.1 Hz), 6.90 (1H, d, *J* = 7.7 Hz), 7.02 (1H, dt, *J* = 0.9, 7.5 Hz), 7.14 (1H, t, *J* = 8.1 Hz), 7.21 (1H, dt, *J* = 0.7, 7.7 Hz), 7.22 (1H, d, *J* = 7.3 Hz), 9.05 (1H, s) ppm.

¹³C-NMR (CDCl₃, TMS, 101 MHz): 180.6, 152.3, 141.7, 134.9, 129.9, 129.6, 127.8, 124.1, 122.2, 119.1, 115.6, 113.7, 109.7, 58.2, 52.9, 48.5, 46.0, 30.3, 26.7, 23.7 ppm.

| Elementary analysis for the Formula C₂₂H₂₆ClN₃O (383.93): | | | | |
|---|---|---|---|---|
| Calculated: | C 68.83, | H 6.83, | Cl 9.23, | N 10.94 %. |
| Found: | C 68.31, | H 6.90, | Cl 9.08, | N 10.77 %. |

### Example 18

### 3-{4-[4-(3-chloro-4-fluorophenyl)-piperazine-1-yl]-butyl}-6-fluoro-1,3-dihydro-2H-indol-2-one monohydrochloride

The title compound is prepared according to Process "B" and applying work-up Procedure 2 using 6-fluoro-3-(4-mesyloxy-butyl)-1,3-dihydro-2*H*-indol-2-one and 1-(3-chloro-4-fluorophenyl)-piperazine as starting compounds.

Melting point 218-224 °C.

IR (KBr): 2577, 1717 (C=O) cm⁻¹.

¹H-NMR (DMSO-*d*₆, TMS, 500 MHz): 1.35-1.24 (2H, m), 1.94-1.74 (4H, m), 3.07 (2H, t, *J* = 7.7 Hz), 3.21 (2H, s), 3.49-3.40 (3H, m), 3.80-3.77 (2H, m), 6.69 (1H, dd, *J*= 2.4, 9.3 Hz), 6.76 (1H, dt, *J* = 2.4, 9.1 Hz), 7.00 (1H, dt, *J* = 3.4, 9.2 Hz), 7.19 (1H, dd, *J* = 2.9, 6.4 Hz), 7.31-7.27 (2H, m), 10.64 (1H, s), 11.29 (1H, sz) ppm.

¹³C-NMR (DMSO-*d*₆, TMS, 125.6 MHz): 22.6, 23.1, 29.6, 44.6, 45.6, 50.4, 55.2, 97. 6 (d, *J* = 26.9 Hz), 107.3 (d, *J* = 22.2 Hz), 116.3 (d, *J* = 6.4 Hz), 117.1 (d, *J* = 21.4 Hz), 117.6, 119.9 (d, *J* = 17.9 Hz), 125.4, 125.4 (d, *J* = 2.6 Hz), 144.5 (d, *J* = 12.4 Hz), 147.2 (d, *J* = 2.1 Hz), 151.5 (d, *J* = 238.8 Hz), 162.1 (d, *J* = 241.0 Hz), 179.3 ppm.

| Elemental analysis for the Formula C₂₂H₂₅Cl₂F₂N₃O (456.37) | | | | |
|---|---|---|---|---|
| Calculated: | C 57.90, | H 5.52, | Cl 15.54, | N 9.21 %. |
| Measured: | C 57.25, | H 5.51, | Cl 15.22, | N 9.01 %. |

### Example 19

### 5-fluoro-3-[4-(4-phenyl-piperazin-1-yl)-butyl]-1,3-dihydro-2H-indol-2-one

The title compound is prepared according to Process "B" and applying work-up Procedure 1 using 5-fluoro-3-(4-mesyloxy-butyl)-1,3-dihydro-2*H*-indol-2-one and 1-phenyl-piperazine as starting compounds.

Melting point, 140-144 °C.

IR (KBr): 3188 (NH), 1705 (C=O) cm⁻¹.

1H-NMR (CDCl₃, TMS, 500 MHz): 1.45-1.34 (2H, m), 1.57-1.52 (2H, m), 1.99-1.95 (2H, m), 2.36 (2H, t, *J* = 7.7 Hz), 2.57 (4H, t, *J =* 5.0 Hz), 3.17 (4H, t, *J* = 5.0 Hz), 3.47 (1H, t, *J* = 6.0 Hz), 6.80 (1H, dd, *J* = 4.3, 8.4 Hz), 6.84 (1H, t, *J* = 7.3 Hz), 6.92-6.87 (3H, m), 6.97 (1H, dd, *J* = 1.8, 8.1 Hz), 7.24 (2H, dd, J= 7.3, 8.8 Hz), 9.52 (1H, s) ppm.

¹³C-NMR (CDCl₃, TMS, 125.6 MHz): 23.6, 26.6, 30.2, 46.5 (d, *J* = 1.7 Hz), 49.0, 53.1, 58.1,110.1 (d, *J* = 8.1 Hz), 112.0 (d, *J* = 24.8 Hz), 114.1 (d, *J* = 23.5 Hz), 115.9, 119.6, 129.0, 131.2 (d, *J* = 8.1 Hz), 137.6 (d, *J* = 2.1 Hz), 151.2, 158.9, 180.7 ppm.

| Elemental analysis for the Formula C₂₂H₂₆FN₃O (367.47) | | | |
|---|---|---|---|
| Calculated: | C 71.91, | H 7.13, | N 11.43 %. |
| Measured: | C 72.17, | H 7.04, | N 11.45 %. |

### Example 20

### 3-{4-[4-(3-chloro-4-fluorophenyl)-piperazine-1-yl]-butyl}-5-fluoro-1,3-dihydro-2H-indol-2-one monohydrochloride

The title compound is prepared acccording to Process "B" and applying work-up Procedure 2, using 5-fluoro-3-(4-mesyloxy-butyl)-1,3-dihydro-2-*H*-indol-2-one and 1-(3-chloro-4-fluomphenyl)-piperazine as starting compounds.

Melting point, 234-237 °C.

IR (KBr): 3143, 2579, 712 (C=O), 1189, 734 cm⁻¹.

¹H-NMR (DMSO-*d*₆, TMS, 500 MHz): 1.33-1.23 (2H, m), 1.95-1.67 (4H, m), 3.78-3.04 (10H, m), 3.51 (1H, t, *J* = 5.8 Hz), 6.83 (1H, dd, *J* = 4.4, 8.4 Hz), 7.03-6.97 (2H, m), 7.22-7.17 (2H, m), 7.29 (1H, t, *J* = 9.1 Hz), 10.45 (1H, s), 11.1 (1H, sz) ppm.

¹³C-NMR (DMSO-*d*₆, TMS, 125.6 Hz): 22.6, 23.2, 29.3, 45.6, 45.7, 50.5, 55.2, 109.9 (d, *J* = 8.1 Hz), 112.1 (d, *J* = 24.8 Hz), 114.0 (d, *J* = 23.5 Hz), 116.3 (d, *J* = 6.8 Hz), 117.1 (d, *J* = 21.8 Hz), 117.6, 119.9 (d, *J* = 17.9 Hz), 131.6 (d, *J* = 8.6 Hz), 139.1 (d, *J* = 1.7 Hz), 147.3, 151.5 (d, *J* = 239.3 Hz), 158.1 (d, *J* = 235.9 Hz), 178.8 ppm.

| Elemental analysis for the Formula C₂₂H₂₅Cl₂F₂N₃O (456.37) | | | | |
|---|---|---|---|---|
| Calculated: | C 57.90, | H 5.52, | Cl 15.54, | N 9.21 %. |
| Measured: | C 57.88, | H 5.63, | Cl 14.94, | N 9.04 %. |

### Example 21

### 3-{4-[4-(3,5-dichlorophenyl)-piperazine-1-yl]-butyl}-1,3-dihydro-2H-indol-2-one monohydrochloride

The title compound is prepared according to Process "B" and applying work-up Procedure 2 using 3-(4-mesyloxy-butyl)-1,3-dihydro-2*H*-indol-2-one and 1-(3,5-dichlorophenyl)-piperazine as starting compounds.

Melting point, 201-205 °C.

IR (KBr): 3416, 3178, 2583, 1706 (C=O), 753 cm⁻¹.

¹H-NMR (DMSO-*d*₆, TMS, 500 MHz): 1.33-1.24 (2H, m), 1.73 (2H, m), 1.94-1.78 (2H, m), 3.04 (4H, sz), 3.25 (2H, sz), 3.46 (3H, t, *J* = 6.0 Hz), 3.92 (2H, sz), 6.83 (1H, d, *J* = 7.7 Hz), 6.95 (1H, d, *J* = 1.5 Hz), 6.95 (1H, dt, *J* = 1.0, 7.5 Hz), 7.04 (1H, d, *J* = 1.4 Hz), 7.18 (1H, tt, *J =* 1.0, 7.6 Hz), 7.27 (1H, d, *J* = 7.3 Hz), 10.40 (1H, s), 11.02 (1H, sz) ppm.

¹³C-NMR (DMSO-*d*₆, TMS, 125.6 MHz): 22.7, 23.1, 29.6, 44.6, 45.0, 50.2, 55.2, 109.4, 113.8, 118.3, 121.4, 124.2, 127.8, 129.7, 134.9, 142.9, 151.5, 178.9 ppm.

| Elemental analysis for the Formula C₂₂H₂₆Cl₃N₃O (454.83) | | | | |
|---|---|---|---|---|
| Calculated: | C 58.10, | H 5.76, | Cl 23.38, | N 9.24 %. |
| Measured: | C 59.10, | H 5.90, | Cl 22.44, | N 9.22 %. |

### Example 22

### 3-{4-[4-(3,4-dichlorophenyl)-piperazine-1-yl]-butyl}-5-fluoro-1,3-dihydro-2H-indol-2-one

The title compound is prepared according to Process "B" and applying workup procedure 1 from 5-fluoro-3-(4-mesyloxy-butyl)-1,3-dihydro-2*H*-indol-2-one and 1-(3,4-dichlorophenyl)-piperazine.

Melting point, 118-120 °C.

IR (KBr): 3200, 1709 (C=O), 835 cm⁻¹.

¹H-NMR (DMSO-*d*₆, TMS, 500 MHz): 1.29-1.21 (2H, m), 1.42 (2H, kv, *J* = 7.2 Hz), 1.93-1.80 (2H, m), 2.25 (2H, t, *J* = 6.1 Hz), 2.42 (4H, t, *J* = 4.9 Hz), 3.13 (4H, t, *J* = 4.9 Hz), 3.48 (1H, t, *J* = 5.7 Hz), 6.79 (1H, dd, *J* = 4.5, 8.4 Hz), 6.9 (1H, dd, *J* = 2.9, 9.0 Hz), 6.99( 1H, dt, *J* = 2.3, 8.9 Hz), 7.10 (1H, d, *J* = 2.8 Hz), 7.16 (1H, dd, *J* = 2.2, 8.4 Hz), 7.37 (1H, d, *J* = 9.0 Hz), 10.35 (1H, d, *J* = 9.0 Hz) ppm.

¹³C-NMR (DMSO-*d*₆, TMS, 125.6 MHz): 23.1, 26.3, 29.5, 45.8, 47.7, 52.5, 57.6, 109.8 (d, *J* = 8.3 Hz), 112.0 (d, *J* = 24.4 Hz), 113.8 (d, *J* = 23.0 Hz), 115.3, 116.2, 119.6, 130.6, 131.6, 131.8 (d, *J* = 8.3 Hz), 139.1 (d, *J* = 1.5 Hz), 150.9, 158.0 (d, *J*= 235.8 Hz), 178.9 ppm.

| Elemental Analysis for the Formula C₂₂H₂₄Cl₂FN₃O (436.36) | | | | |
|---|---|---|---|---|
| Calculated: | C 60.56, | H 5.54, | Cl 16.25, | N 9.63 %. |
| Measured: | C 60.71, | H 5.64, | Cl 16.14, | N 9.72 %. |

### Example 23

### 3-[4-(4-phenyl-piperazin-1-yl)-butyl]-1,3-dihydro-2H-indol-2-one

The title compound is prepared according to Process "B" and applying workup Procedure 1 from 3-(4-mesyloxy-butyl)-1,3-dihydro-2*H*-indol-2-one and 1-phenylpiperazine.

Melting point, 110-113 °C.

IR (KBr): 3191, 1705 (C=O) cm⁻¹.

¹H-NMR (DMSO-*d*₆, TMS, 500 MHz): 1.31-1.24 (2H, m), 1.43 (2H, kv, *J* = 7.2 Hz), 1.82-1.79 (1H, m), 1.91-1.81 (1H, m), 2.25 (2H, t, *J* = 7.3 Hz), 2.43 (4H, t, *J* = 5.0 Hz), 3.07 (4H, t, *J* = 4.9 Hz), 3.42 (1H, t, *J* = 4.9 Hz), 6.76 (1H, t, *J* = 7.2 Hz), 6.82 (1H, d, *J* = 7.7 Hz), 6.90 (1H, dd, *J*= 1.0, 7.8 Hz), 6.94 (1H, dt, *J* = 1.0, 7.6 Hz), 7.21-7.14 (3H, m), 7.24 (1H, d, *J* = 7.3 Hz), 10.35 (1H, s) ppm.

¹³C-NMR (DMSO-*d*₆, TMS, 125.6 MHz): 23.3, 26.4, 29.9, 45.3, 48.3, 52.9, 57.8, 109.3, 115.4, 118.8, 121.3, 124.1, 127.7, 129.0, 129.9, 142.9, 151.2, 179.1 ppm.

| Elemental Analysis for the Formula C₂₂H₂₇N₃O (349.48) | | | |
|---|---|---|---|
| Calculated: | C 75.61, | H 7.79, | N 12.02 %. |
| Measured: | C 74.53, | H 7.81, | N 11.81 %. |

### Example 24

### 6-fluoro-3-{4-[4-(4-fluorophenyl)-piperazin-1-yl]-butyl}-1,3-dihydro-2H-indol-2-one monohydrochloride

The title compound is prepared using Process "B" and applying work-up Procedure 2, using 6-fluoro-3-(4-mesyloxy-butyl)-1,3-dihydro-2*H*-indol-2-one and 1-(4-fluorophenyl)-piperazine as starting compounds.

Melting point, 184-189 °C.

IR (KBr): 3125, 1717 (C=O), 1512 cm⁻¹.

¹H-NMR (DMSO-*d*₆, TMS, 500 MHz): 1.32-1.24 (2H, m), 1.92-1.73 (4H, m), 3.17-3.06 (6H, m), 3.52-3.45 (3H, m), 3.70-3.68 (2H, m), 7.31-6.66 (7H, m), 10.62 (1H, s), 11.2 (1H, sz) ppm.

¹³C-NMR (DMSO-*d*₆, TMS, 125.6 MHz): 22.6, 23.1, 29.6, 44.6, 46.2, 50.6, 55.1, 97.6 (d, *J* = 26.9 Hz), 107.3 (d, *J* = 22.0 Hz), 115.6 (d, *J* = 22.0 Hz), 118.0, 125.3, 125.4, 144.5, 146.6, 155.8, 157.7, 161.1, 163.0, 179.3 ppm.

| Elemental Analysis | | | | |
|---|---|---|---|---|
| C₂₂H₂₆ClF₂N₃O (421.92) | | | | |
| Calculated: | C 62.63, | H 6.21, | Cl 8.40, | N 9.96 %. |
| Measured: | C 62.37, | H 6.31, | Cl 8.41, | N 9.78 %. |

## Claims

1. 3-Alkyl indol-2-one derivatives of the general Formula (I), 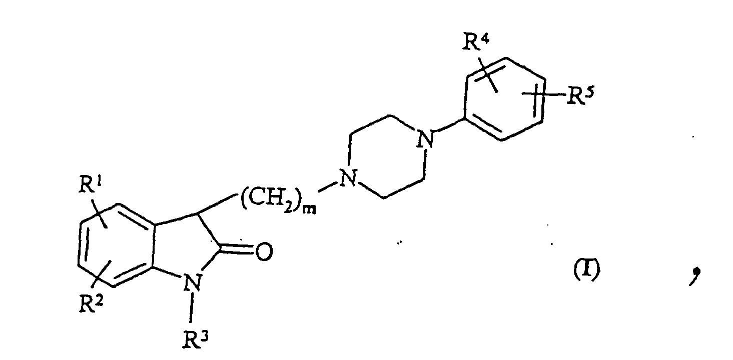 wherein
R¹ represents hydrogen, halogen, alkyl having 1 to 7 carbon atom(s);
R² and R³ represent hydrogen;
R⁴ is hydrogen or halogen;
R⁵ stands for halogen or trifluoromethyl
m is 4;
and pharmaceutically acceptable acid addition salts thereof.

2. 3-Alkyl indol-2-one derivatives according to claim 1 of the general Formula (I),
wherein
R¹ represents hydrogen or halogen;
R² R³ and R⁴ denote hydrogen;
R⁵ is halogen;
m is 4;
and pharmaceutically acceptable acid addition salts thereof.

3. 3-{4-[4-(4-Chlorophenyl)-piperazin-1-yl]-butyl}--6-fluoro-1,3-dihydro-2H-indol-2-one, and pharmaceutically acceptable acid addition salts thereof.

4. 3-{4-[4-(4-Fluorophenyl)-piperazin-1-yl]-butyl}-1,3--dihydro-2H-indol-2-one, and pharmaceutically acceptable acid addition salts thereof.

5. 3-{4-[4-(4-Chlorophenyl)-piperazin-1-yl]-butyl}-1,3--dihydro-2H-indol-2-one, and pharmaceutically acceptable acid addition salts thereof.

6. 3-{4-[4-(3-Chlorophenyl)-piperazin-1-yl]-butyl}-1,3--dihydro-2H-indol-2-one, and pharmaceutically acceptable acid addition salts thereof.

7. 3-{4-[4-(3-Chlorophenyl)-piperazin-1-yl]-butyl}--5-methyl-1,3-dihydro-2H-indol-2-one, and pharmaceutically acceptable acid addition salts thereof.

8. 3-{4-[4-(4-Chlorophenyl)-piperazin-1-yl]-butyl}--5-methyl-1,3-dihydro-2H-indol-2-one, and pharmaceutically acceptable acid addition salts thereof.

9. 3-{4-[4-(4-Chloro-3-trifluoromethyl-phenyl)-piperazin--1-yl]-butyl}-1,3-dihydro-2H-indol-2-one, and pharmaceutically acceptable acid addition salts thereof.

10. Pharmaceutical compositions comprising as active ingredient at least one compound of the general Formula (I) according to any of claims 1 to 9 or a pharmaceutically acceptable acid addition salt thereof in admixture with one or more conventional carrier(s) or auxiliary agent(s).

11. Pharmaceutical compositions according to claim 10 useful for the treatment or prophylaxis of central nervous disorders, particularly depression, anxiety, compulsive disease, panic disease, social phobia, schizophrenia, mood disorders, mania, mental decline, stroke, cell death in certain areas of the central nervous system, neurodegeneration followed by mental decline, Alzheimer's disease, dementia, post-traumatic disease, stress disease, disorders of the cardiovascular system, particularly hyper-tension.

12. Process for the preparation of compounds of the general Formula (I) as specified in claim 1,
which comprises
a./ reacting a compound of the general Formula (II), 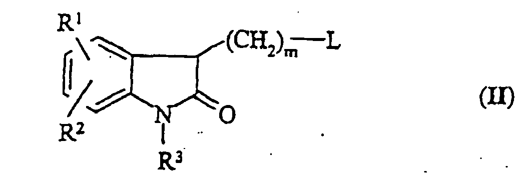 wherein L represents hydroxy, with an aryl-sulfonyl chloride or a straight or branched chain alkylsulfonyl chloride having 1 to 7 carbon atom(s) in the presence of an organic base, and reacting the thus-obtained compound of the general Formula (II), wherein L represents aryl- or alkylsulfonyloxy, with a pyridine derivative of the general Formula (III), 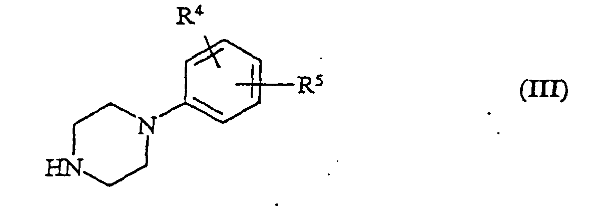 wherein R⁴ and R⁵ are as stated above, in the presence of an acid binding agent, or
b./ reacting a compound of the general Formula (V), 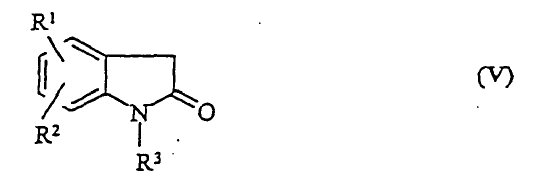 wherein R¹, R² and R³ are as stated above, with a compound of the general Formula (VI), 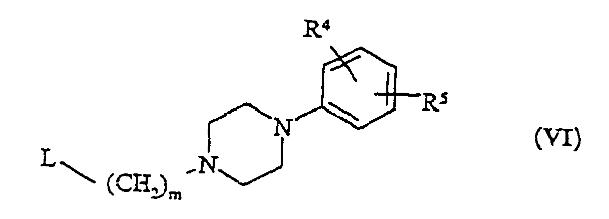 wherein R⁴, R⁵ and m are as stated above and L is an alkylsulfonyloxy or arylsulfonyloxy group or a halogen atom, in the presence of a strong base.

13. Use of the 3-alkyl indol-2-one derivatives of the general Formula (I) according to any of claims 1 to 9 for preparing a medicament.

14. Use of the compounds according to any of claims 1 to 9 of the general Formula (I) or pharmaceutically acceptable acid addition salts thereof for the manufacture of medicaments suitable for the treatment or prophylaxis of central nervous disorders, particularly depression, anxiety, compulsory disease, panic disease, social phobia, schizophrenia, mood disorders, mania, mental decline, stroke, cell death in certain parts of the central nervous system, neurodegeneration followed by mental decline, Alzheimer's disease, dementia, post-traumatic disease, stress disease, disorders of the cardiovascular system, particularly hyper-tension.

15. A process for the preparation of a pharmaceutical composition for the treatment or prophylaxis of central nervous disorders, particularly depression, anxiety, compulsory disease, panic disease, social phobia, schizo-phrenia, mood disorders, mania, mental decline, stroke, cell death in certain parts of the central nervous system, neurodegeneration followed by mental decline, Alzheimer's disease, dementia, post-traumatic disease, stress disease, disorders of the cardiovascular system, particularly hypertension, which comprises admixing at least one compound of the general Formula (I) according to any of claims 1 to 9 or a pharmaceutically acceptable acid addition salt thereof with a pharmaceutical carrier and optionally other auxiliary agent and bringing the mixture to galenic form.

## Patentansprüche

1. 3-Alkylindol-2-on-Derivate der allgemeinen Formel (I): 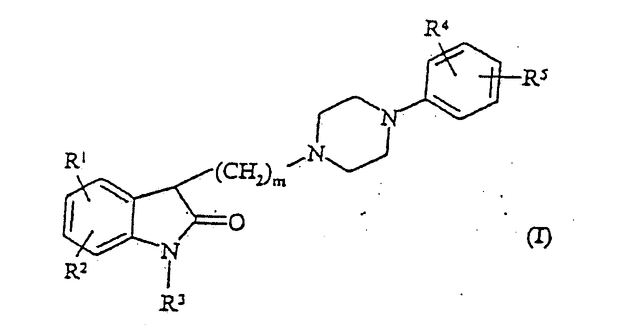 worin
R¹ Wasserstoff, Halogen, Alkyl mit 1 bis 7 Kohlenstoffatomen repräsentiert;
R² und R³ Wasserstoff repräsentieren;
R⁴ Wasserstof oder Halogen ist;
R⁵ für Halogen oder Trifluormethyl steht;
m 4 ist;
und pharmazeutisch akzeptable Säureadditionssalze davon.

2. 3-Alkylindol-2-on-Derivate gemäß Anspruch 1 der allgemeinen Formel (I):
worin
R¹ Wasserstoff oder Halogen repräsentiert;
R², R³ und R⁴ Wasserstoff bezeichnen;
R⁵ Halogen ist;
m 4 ist;
und pharmazeutisch akzeptable Säureadditionssalze davon.

3. 3-{4-[4-(4-Chlorphenyl)-piperazin-1-yl]-butyl}-6-fluor-1,3-dihydro-2H-indol-2-on und pharmazeutisch akzeptable Säureadditionssalze davon.

4. 3-{4-[4-(4-Fluorphenyl)-piperazin-1-yl]-butyl}-1,3-dihydro-2H-indol-2-on und pharmazeutisch akzeptable Säureadditionssalze davon.

5. 3-{4-[4-(4-Chlorphenyl)-piperazin-1-yl]-butyl}-1,3-dihydro-2H-indol-2-on und pharmazeutisch akzeptable Säureadditionssalze davon.

6. 3-{4-[4-(3-Chlorphenyl)-piperazin-1-yl]-butyl}-1,3-dihydro-2H-indol-2-on und pharmazeutisch akzeptable Säureadditionssalze davon.

7. 3-{4-[4-(3-Chlorphenyl)-piperazin-1-yl]-butyl}-5-methyl-1,3-dihydro-2H-indol-2-on und pharmazeutisch akzeptable Säureadditionssalze davon.

8. 3-{4-[4-(4-Chlorphenyl)-piperazin-1-yl]-butyl}-5-methyl-1,3-dihydro-2H-indol-2-on und pharmazeutisch akzeptable Säureadditionssalze davon.

9. 3-{4-[4-(4-Chlor-3-trifluormethyl-phenyl)-piperazin-1-yl]-butyl}-1,3-dihydro-2H-indol-2-on und pharmazeutisch akzeptable Säureadditionssalze davon.

10. Pharmazeutische Zusammensetzungen, umfassend als aktiven Bestandteil mindestens eine Verbindung der allgemeinen Formel (I) gemäß einem der Ansprüche 1 bis 9 oder ein pharmazeutisch akzeptables Säureadditionssalz davon in Vermischung mit einem oder mehreren herkömmlichen Träger(n) oder Hilfsstoff(en).

11. Pharmazeutische Zusammensetzungen gemäß Anspruch 10, die für die Behandlung oder Prophylaxe von Störungen des zentralen Nervensystems, insbesondere Depression, Angstgefühl, Zwangsleiden, Panikleiden, Sozialphobie, Schizophrenie, Gemütsstörungen, Manie, geistigem Verfall, Schlaganfall, Zelltod in bestimmten Bereichen des zentralen Nervensystems, Neurodegeneration, gefolgt von geistigem Verfall, Alzheimer-Krankheit, Demenz, posttraumatischer Erkrankung, Stresserkrankung, Störungen des kardiovaskulären Systems, insbesondere von Bluthochdruck, nützlich sind.

12. Verfahren für die Herstellung von Verbindungen der allgemeinen Formel (I), wie in Anspruch 1 ausgeführt, umfassend:
a) Umsetzen einer Verbindung der allgemeinen Formel (II): 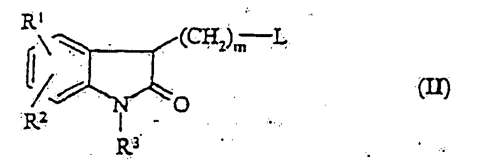 worin L Hydroxy repräsentiert, mit einem Arylsulfonylchlorid oder einem gerad- oder verzweigtkettigen Alkylsulfonylchlorid mit 1 bis 7 Kohlenstoffatomen in Gegenwart einer organischen Base und Reagierlassen der so erhaltenen Verbindung der allgemeinen Formel (II), worin L Aryl- oder Alkylsulfonyloxy repräsentiert, mit einem Pyridinderivat der allgemeinen Formel (III): 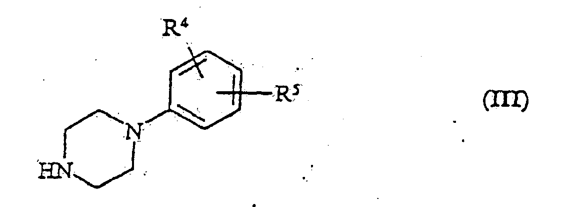 worin R⁴ und R⁵ wie oben angegeben sind, in Gegenwart eines Säurebindungsmittels, oder
b) Umsetzen einer Verbindung der allgemeinen Formel (V): 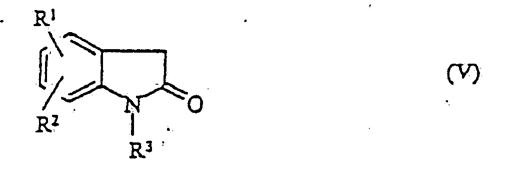 worin R¹, R² und R³ wie oben angegeben sind, mit einer Verbindung der allgemeinen Formel (VI): 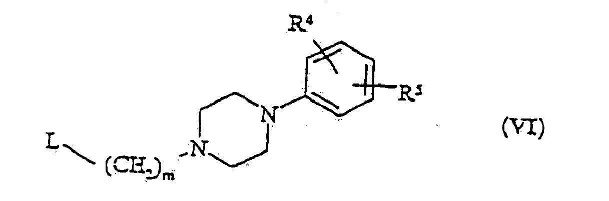 worin R⁴, R⁵ und m wie oben angegeben sind und L eine Alkylsulfonyloxy- oder Arylsulfonyloxygruppe oder ein Halogenatom ist, in Gegenwart einer starken Base.

13. Verwendung der 3-Alkyl-indol-2-on-Derivate der allgemeinen Formel (I) gemäß einem der Ansprüche 1 bis 9 für die Herstellung eines Medikaments.

14. Verwendung der Verbindungen gemäß einem der Ansprüche 1 bis 9 der allgemeinen Formel (I) oder von pharmazeutisch akzeptablen Säureadditionssalzen davon für die Herstellung von Medikamenten, die für die Behandlung oder Prophylaxe von Störungen des zentralen Nervensystems, insbesondere Depression, Angstgefühl, Zwangsleiden, Panikleiden, Sozialphobie, Schizophrenie, Gemütsstörungen, Manie, geistigem Verfall, Schlaganfall, Zelltod in bestimmten Teilen des zentralen Nervensystems, Neurodegeneration, gefolgt von geistigem Verfall, Alzheimer-Krankheit, Demenz, posttraumatischer Erkrankung, Stresserkrankung, Störungen des kardiovaskulären Systems, insbesondere von Bluthochdruck, geeignet sind.

15. Verfahren für die Herstellung einer pharmazeutischen Zusammensetzung für die Behandlung oder Prophylaxe von Störungen des zentralen Nervensystems, insbesondere Depression, Angstgefühl, Zwangsleiden, Panikleiden, Sozialphobie, Schizophrenie, Gemütsstörungen, Manie, geistigem Verfall, Schlaganfall, Zelltod in bestimmten Teilen des zentralen Nervensystems, Neurodegeneration, gefolgt von geistigem Verfall, Alzheimer-Krankheit, Demenz, posttraumatischer Erkrankung, Stresserkrankung, Störungen des kardiovaskulären Systems, insbesondere von Bluthochdruck, umfassend das Vermischen mindestens einer Verbindung der allgemeinen Formel (I) gemäß einem der Ansprüche 1 bis 9 oder eines pharmazeutisch akzeptablen Säureadditionssalzes davon mit einem pharmazeutischen Träger und gegebenenfalls einem anderen Hilfsstoff und Bringen der Mischung in eine galenische Form.

## Revendications

1. Dérivés de 3-alkyl indol-2-one de formule générale (I), 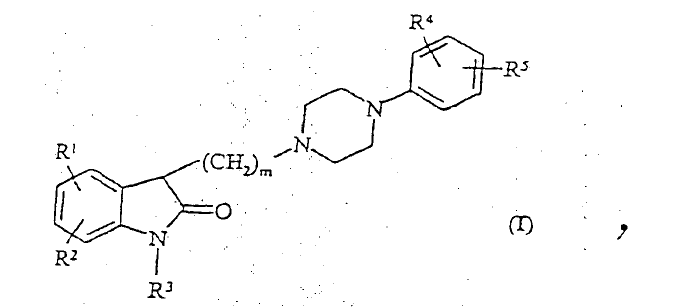 dans laquelle
R¹ représente de l'hydrogène, un halogène, un alkyle ayant 1 à 7 atome(s) de carbone ;
R² et R³ représentent de l'hydrogène ;
R⁴ est de l'hydrogène ou un halogène ;
R⁵ représente un halogène ou un trifluorométhyle
m est 4 ;
et les sels d'addition pharmaceutiquement acceptables de ceux-ci avec un acide.

2. Dérivés de 3-alkyl indol-2-one selon la revendication 1 de formule générale (I), dans laquelle
R¹ représente de l'hydrogène ou un halogène ;
R², R³ et R⁴ représentent de l'hydrogène ;
R⁵ est un halogène ;
m est 4 ;
et les sels d'addition pharmaceutiquement acceptables de ceux-ci avec un acide.

3. 3-{4-[4-(4-Chlorophényl)-pipérazin-1-yl]-butyl}-6-fluoro-1,3-dihydro-2H-indol-2-one, et les sels d'addition pharmaceutiquement acceptables de celle-ci avec un acide.

4. 3-{4-[4-(4-Fluorophényl)-pipérazin-1-yl]-butyl}-1,3-dihydro-2H-indol-2-one, et les sels d'addition pharmaceutiquement acceptables de celle-ci avec un acide.

5. 3-{4-[4-(4-Chlorophényl)-pipérazin-1-yl]-butyl}-1,3-dihydro-2H-indol-2-one, et les sels d'addition pharmaceutiquement acceptables de celle-ci avec un acide.

6. 3-{4-[4-(3-Chlorophényl)-pipérazin-1-yl]-butyl}-1,3-dihydro-2H-indol-2-one, et les sels d'addition pharmaceutiquement acceptables de celle-ci avec un acide.

7. 3-{4-[4-(3-Chlorophényl)-pipérazin-1-yl]-butyl}-5-méthyl-1,3-dihydro-2H-indol-2-one, et les sels d'addition pharmaceutiquement acceptables de celle-ci avec un acide.

8. 3-{4-[4-(4-Chlorophényl)-pipérazin-1-yl]-butyl}-5-méthyl-1,3-dihydro-2H-indol-2-one, et les sels d'addition pharmaceutiquement acceptables de celle-ci avec un acide.

9. 3-{4-[4-(4-Chloro-3-trifluorométhyl-phényl)-pipérazin-1-yl]-butyl}-1,3-dihydro-2H-indol-2-one, et les sels d'addition pharmaceutiquement acceptables de celle-ci avec un acide.

10. Compositions pharmaceutiques comprenant, en tant qu'ingrédient actif, au moins un composé de formule générale (I) selon l'une quelconque des revendications 1 à 9 ou un sel d'addition pharmaceutiquement acceptable de celui-ci avec un acide, en mélange avec un ou plusieurs véhicule(s) ou agent(s) auxiliaire(s) classique(s).

11. Compositions pharmaceutiques selon la revendication 10, utiles pour le traitement ou la prophylaxie de troubles nerveux centraux, en particulier une dépression, une anxiété, une maladie compulsive, une maladie panique, une phobie sociale, une schizophrénie, des troubles de l'humeur, une manie, un déclin mental, une attaque cérébrale, une mort cellulaire dans certaines zones du système nerveux central, une neurodégénérescence suivie par un déclin mental, une maladie d'Alzheimer, une démence, une maladie post-traumatique, une maladie de stress, des troubles du système cardiovasculaire, en particulier l'hypertension.

12. Procédé pour la préparation de composés de formule générale (I) telle que spécifiée dans la revendication 1, qui comprend
a. la réaction d'un composé de formule générale (II), 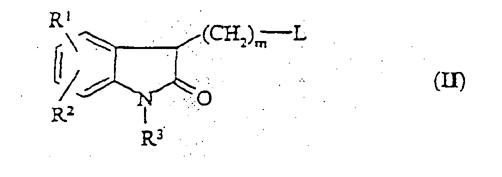 dans laquelle L représente un hydroxy, avec un chlorure d'aryl-sulfonyle, ou un chlorure d'alkyl-sulfonyle à chaîne linéaire ou ramifiée ayant 1 à 7 atome(s) de carbone, en présence d'une base organique, et la réaction du composé de formule générale (II) ainsi obtenu, dans lequel L représente un aryl- ou alkylsulfonyloxy, avec un dérivé de pyridine de formule générale (III), 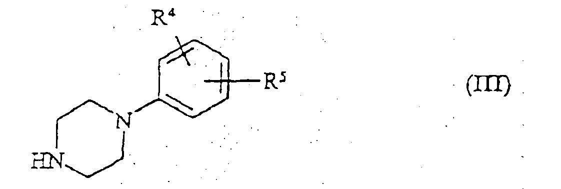 dans laquelle R⁴ et R⁵ sont tels que décrits ci-dessus, en présence d'un agent de liaison à un acide, ou
b. la réaction d'un composé de formule générale (V), 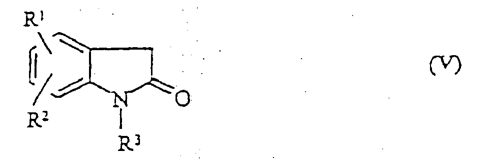 dans laquelle R¹, R² et R³ sont tels que décrits ci-dessus, avec un composé de formule générale (VI), 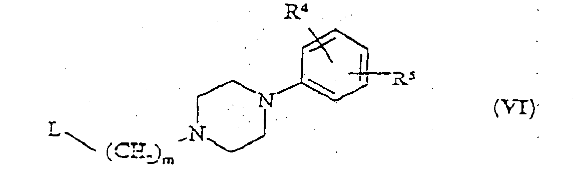 dans laquelle R⁴, R⁵ et m sont tels que décrits ci-dessus et L est un groupe alkylsulfonyloxy ou arylsulfonyloxy ou un atome d'halogène, en présence d'une base forte.

13. Utilisation des dérivés de 3-alkyl indol-2-one de formule générale (I) selon l'une quelconque des revendications 1 à 9, pour préparer un médicament.

14. Utilisation des composés selon l'une quelconque des revendications 1 à 9 de formule générale (I) ou de sels d'addition pharmaceutiquement acceptables de ceux-ci avec un acide, pour la fabrication de médicaments convenant au traitement ou à la prophylaxie de troubles nerveux centraux, en particulier une dépression, une anxiété, une maladie compulsive, une maladie panique, une phobie sociale, une schizophrénie, des troubles de l'humeur, une manie, un déclin mental, une attaque cérébrale, une mort cellulaire dans certaines zones du système nerveux central, une neurodégénérescence suivie par un déclin mental, une maladie d'Alzheimer, une démence, une maladie post-traumatique, une maladie de stress, des troubles du système cardiovasculaire, en particulier l'hypertension.

15. Procédé pour la préparation d'une composition pharmaceutique pour le traitement ou la prophylaxie de troubles nerveux centraux, en particulier une dépression, une anxiété, une maladie compulsive, une maladie panique, une phobie sociale, une schizophrénie, des troubles de l'humeur, une manie, un déclin mental, une attaque cérébrale, une mort cellulaire dans certaines zones du système nerveux central, une neurodégénérescence suivie par un déclin mental, une maladie d'Alzheimer, une démence, une maladie post-traumatique, une maladie de stress, des troubles du système cardiovasculaire, en particulier l'hypertension, qui comprend le mélange d'au moins un composé de formule générale (I) selon l'une quelconque des revendications 1 à 9 ou d'un sel d'addition pharmaceutiquement acceptable de celui-ci avec un acide avec un véhicule pharmaceutique et facultativement un autre agent auxiliaire, et la mise du mélange sous forme galénique.
